# EUROPEAN PATENT SPECIFICATION

(11) **EP 1 993 452 B1**
(45) Date of publication and mention of the grant of the patent: **12.07.2017**
(21) Application number: 07752363.7
(22) Date of filing: 05.03.2007
(51) Int. Cl.: A61B 17/30, A61B 1/012, A61B 17/122, A61B 17/128

(54) **A CLIP DEVICE AND A PROTECTIVE CAP FOR DRAWING THE TARGET TISSUE INTO IT BEFORE THE CLIP IS DEPLOYED**
KLAMMERVORRICHTUNG UND SCHUTZKAPPE ZUM HINEINZIEHEN DES ZIELGEWEBES VOR EINSATZ DER KLAMMER
CLIP ET COIFFE PROTECTRICE, ET PROCEDES LES UTILISANT AVEC UN ENDOSCOPE POUR LA SAISIE D'UN TISSU PAR VOIE ENDOSCOPIQUE

(30) Priority: 10.03.2006 US 780994 P
(43) Date of publication of application: 26.11.2008
(73) Proprietor: Cook Medical Technologies LLC, Bloomington, IN 47404 (US)
(72) Inventor: SOETIKNO, Roy, M., Union City, CA 94587 (US); SURTI, Vihar, C., Winston-Salem, NC (US)
(74) Representative: Garratt, Peter Douglas
(86) International application number: PCT/US2007/005653
(87) International publication number: WO 2007/106342

(56) References cited:
- WO-A-03/088850
- US-A- 6 120 434
- US-A1- 2002 133 178
- US-A1- 2004 193 184
- US-A1- 2005 261 711

## Description

### TECHNICAL FIELD

The present invention relates to a clip device and more specifically to a protective cap and clip device for delivering a clip that can be used to cause hemostasis of blood vessels along the gastrointestinal tract, or that can be used as an endoscopic tool for holding tissue and the like.

In describing embodiments of the present invention, the following disclosure also describes methods of grasping tissue endoscopically. More particularly, there are described methods of using a protective cap and a clip device with an endoscope to grasp tissue endoscopically for performing hemostasis, tissue marking, endoscopic mucosal resection, tissue ligation, and a number of other applications relating to colorectal medical procedures or gastrointestinal bleeding and the like.

### BACKGROUND OF THE INVENTION

Gastrointestinal bleeding presents a common emergency that might occur during gastroenterology practice. If left untreated, this common and serious condition sometimes can lead to fatal results. While this problem has prompted a few endoscopic therapeutic approaches to achieve hemostasis, such as injecting sclerosing agents and using contact thermo-coagulation techniques, such approaches occasionally allow continued bleeding for many patients. Corrective surgery then becomes necessary. Because surgery requires invasive techniques sometimes associated with a high mortality rate or other undesirable side effects, there exists a need for highly effective less invasive procedures.

In addition, conventional mechanical haemostatic devices are sometimes used by physicians, operators, gastroenterologists, or other healthcare professionals ("gastroenterologists") in various parts of the body, including gastrointestinal applications. Such haemostatic devices typically include clamps, clips, staples, sutures, and other similar devices that are able to apply a sufficient constrictive force to blood vessels so as to limit or interrupt blood flow. One of
the problems associated with using conventional haemostatic devices, however, is that they can only be delivered using rigid shafted instruments via incision or trocar cannula. Moreover, none of the conventional haemostatic devices are strong enough to cause permanent hemostasis.

Clip devices address those situations where conventional endoscopic therapeutic approaches and mechanical haemostatic devices for controlling gastrointestinal bleeding fail. Examples of suitable clip devices are available through Wilson-Cook Medical Incorporated. Gastroenterologists introduce a clip into a body cavity through a flexible endoscope working channel to grasp living tissue within the body cavity for performing hemostasis, tissue marking, endoscopic mucosal resection, and tissue ligation. Gastroenterologists are now using clips this way in many other applications relating to gastrointestinal bleeding, such as peptic ulcers, Mallory- Weiss tears, Dieulafoy's lesions, angiomas, post-papillotomy bleeding, and small varices with active bleeding, as well as in colorectal procedures. Further examples of clip devices are provided in WO03/088850, over which Claim 1 is characterised. This document discloses a clip device for hemostasis that includes an outer sheath that may be inserted into a body cavity via a channel of an endoscope that has been previously inserted into the body cavity. An operating wire is slidably inserted into an inner sheath, which in turn is separately advanceable and retractable within the outer sheath. The operating wire has a distal end portion and a retainer attached to the distal end portion of the operating wire. The clip device further includes a clip having a proximal end portion and provided with a number of arms having a tendency to be in an open position. A first retainer is attached to the distal end of the clip and is matingly received by a second retainer provided on the operating wire. A clip sliding ring is provided for closing the arm portions of the clip.
The document describes the device being used in the following manner: while the body cavity is observed via the endoscope, the distal end portion of the outer sheath is guided to a part to be treated, leaving the distal end of the endoscope in the process; the outer sheath is then pulled toward the proximal end (i. e., retracted) to expose the clip and the distal end portion of the inner sheath; the inner sheath is then advanced toward the clip causing the sliding ring to slide toward the arms of the clip causing the arms to close on the target tissue; the inner sheath is then retracted and when the distal end of the inner sheath passes the first and second retainers, they detach and release from each other and the clip is left inside the body cavity, holding the tissue.
Reference is further directed to US2002/133178, which discloses an apparatus for ligating living tissues that includes an introducing tube capable of being inserted into the channel of an endoscope, at least two or more manipulating wires movably inserted into the introducing tube, and at least two or more clips having a proximal end portion, a pinch section being formed at a distal end of an arm section extending from the proximal end. A plurality of clips is arranged in series in the introducing tube so as to engage the clip and the manipulating wire with each other, respectively. A distal end tip is provided at a distal end portion of the introducing tube.
In use, the introducing tube is introduced via the channel of an endoscope and is advanced so that its distal end portion is located in close proximity to a clipping target tissue. A clip is then protruded from the distal end tip and the arm sections open. Then, while the pinch sections of the arms are pushed against the clipping target tissue, the manipulating wire is retracted so that the proximal end portion of the clip is introduced into the distal end tip, and projections provided at the arm sections of the clip are engaged with a distal end face of the distal end tip. When the manipulating wire is further retracted, the proximal end portion of the clip is plastically deformed and the pinch sections of the arms are closed, whereby the clipping target tissue is pinched. Then, the manipulating wire is further retracted, which causes the manipulating wire to separate from the clip, with the clip being retained within the body cavity.

While U.S. Pat. No. 5,766,189 describes one proposed solution and shows a device that gastroenterologists may use to deliver a detachable clip having a pair of open arms that close about a blood vessel, one problem with using this clip device and other similar clip devices having only two arms is that gastroenterologists need to rotate the clip in order to grasp properly the area to be clipped. Rotating the operating wire through the bends of the tube(s) used to deliver the clip, however, often hinders or complicates the procedure.

Accordingly, there is a need for a clip device and method of delivering the clip to the target area and using the clip without having to rotate the clip to a desired orientation.

It is proposed to solve this problem by providing a detachable clip having at least three arms.

Another problem often encountered with using conventional clip devices is the difficulty in securing the clip device to the delivery apparatus prior to reaching the target area within the patient. Moreover, the gastroenterologist needs to be
able to quickly and easily release the clip device from the delivery apparatus once the clip has been attached to the target site.

It is proposed to solve this and other problems by providing a detachable clip having a retainer system and a sliding ring that secures the retainer system.

Turning to another problem to be solved, gastroenterologists often encounter blood or other bodily fluids that obscure the surgical site when using conventional clip devices. These fluids increase the difficulty in properly positioning the device and grasping the area to be clipped. For example, when attempting to clip a bleeding vessel, blood typically fills the gastroenterologists' working space, thereby impeding the gastroenterologists' ability to locate and/or clip the vessel. As a result, gastroenterologists' usually flush the surgical site with saline to wash away any blood or other bodily fluids obstructing their visibility of the site, but they typically perform this flushing procedure by using a separate catheter after inserting the catheter into the patient and directing the catheter to the surgical site. In addition to the need for a separate catheter, the procedure often results in delays in clipping the vessel, because additional time is needed for inserting and positioning the catheter.

It is proposed to solve this and other problems by providing a clip delivery device that optionally integrates a flushing feature.

Conventional delivery apparatus allow placement of clips through an endoscope working channel. For instance, conventional delivery apparatus utilize an elongate wire extending the length of the working channel to detachably connect at the distal end to a clip with arms that open and close to engage tissue. The clip-wire assembly is slideably contained within two other coaxial sheaths and a holding tube - all of which must be received within the endoscope working channel. For example, a hollow coil sheath extends the length of the working channel, which sheath slideably receives the wire assembly, wherein the clip arms extend distal of a distal end of the hollow coil sheath. A releasable holding tube
circumferentially attaches to the outside of the hollow coil sheath distal end. An outer tubular sheath extends the length of the working channel and slideably receives the hollow coil sheath-clip-wire assembly such that the outer tubular sheath distal end extends over the clip arms with the arms in a closed position. The outer tube sheath is pulled proximally in order to expose the clip arms, which expand to an open, engaging position. In this state, the clip arms may receive the object tissue, and the clip-wire assembly pulled proximally so that the clip arms are moved to a closed position by the distal end of the hollow coil sheath and constrained in a closed position by the holding tube. The wire is disengaged from the clip, and the clip (constrained by the holding tube) are left in the patient's body.

One problem with these conventional through-the-scope clip delivery apparatus is that the delivery apparatus (e.,g., clip-wire assembly, holding tube, hollow coil sheath, and outer tubular sheath distal end) may be too large to be placed through smaller endoscope working channels. Another problem is that two coaxial tubes are needed, and still another problem is that the holding tube must have an inner diameter that is large enough to attach to the outside of the hollow coil sheath, and the outer diameter of the outer tubular sheath needs to be greater than the outer diameter of the holding tube. Yet another problem is that the conventional coaxial tubes are prone to kinking, buckling, bending, and/or bowing that result from passing the delivery apparatus through the working channel of an endoscope, pulling the wire proximally, or sliding one tube relative to the other tube.

The present invention solves these and other problems by using clip devices with a protective cap detachably secured externally over a distal end portion of the endoscope distal insert. Because the protective cap does not pass through the endoscope working channel, the clip device delivery apparatus of the present invention may be used with a smaller inner diameter endoscope working channel. Also, one tube optionally may be eliminated (e.g., using either the endoscope distal insert as the "outer tube" or the externally attached protective cap as the "outer tube"), thereby allowing the clip device to pass through a smaller endoscope working channel and reducing manufacturing costs. Furthermore, eliminating one or more tubes reduces the tendency of the tube(s) to kink, buckle, bend, and/or bow when used in an endoscope working channel.

The following disclosure describes a method for achieving an even faster, easier, and equally efficient, safe, and reliable way to isolate the target tissue and to perform hemostasis, tissue marking, endoscopic mucosal resection, tissue ligation, and many other applications related to colorectal medical procedures or gastrointestinal bleeding and the like (individually and collectively, "hemostasis," "haemostatic," and variants thereof). The method teaches using clip devices together with a protective cap detachably secured to a distal end portion of an endoscope insert, and using suction from the conventional endoscope to draw the target tissue into the protective cap.

Furthermore, the following disclosure describes a method for helping to ensure that an endoscope camera lens has a substantially unobstructed view of the target tissue. The method teaches using clip devices together with a protective cap that is detachably secured to a distal end of an endoscope insert. Because the cap extends distally to the camera lens, the cap helps to keep the camera lens spaced from and relatively free of blood and other bodily fluids.
A variety of endoscope constructions and component parts is known in the art. For example, US 6,120,434 discloses an endoscopic sheath comprising a tubular sheath section which can be inserted into a patient's body. The sheath section is composed of a sheath body, formed of a rigid hollow member, such as a stainless-steel pipe, and a transparent cap attached to the distal end portion of the sheath body. The transparent cap may be a hollow cylinder, formed integrally with a ring-shaped ridge on its inner peripheral surface. This ring-shaped ridge projects, and serves as a stopper which abuts against the distal end of the rigid endoscope. The ring-shaped ridge is situated in a position such that at least part of the distal end portion of the transparent cap is within the view range of the rigid endoscope. Tissues may therefore be observed through the distal opening of the transparent cap, so that a subject region to be observed through the opening cannot be pressed by the sheath section. The document does not however mention the possibility of using such an endoscopic sheath with a clip device.
Reference is further directed to US2002/035311, which discloses an endoscope for use in endoscopic mucosectomy, whose tip portion has a tubular hood. During use, the mucosa of a diseased part in a body cavity is aspirated into the tip hood so that it becomes like a "polyp" in shape and its base is cut off with a high frequency snare. The document teaches that a regulating portion is provided in a position intermediate between the distal end and the bottom of the tip hood so as to ensure that the mucous membrane aspirated from the distal end of the tip hood toward an aspiration port will not get to the viewing window of the endoscope and thereby interfere with the visual field. The document does not however mention the possibility of using such a hood with a clip device.
Reference is still further directed to EP1 508 294A, which discloses an endoscope with a hood that is integrally or detachably provided at the distal end of its insertion portion. This insertion portion includes: an observation window; an illumination port; air/water feed opening; and forceps opening, on the distal end portion thereof. The hood maintains a suitable distance between the observation window and the tissue portion which is to be observed, so as to obtain clear endoscopic images. However, the hood is also formed with a suitable thickness, a suitable length extending from the distal end surface of the insertion portion up to the front surface of the endoscope hood, and so forth, such that it does not obstruct the field of view of the observation optical system, leading to shading. It should further be noted that, while the document mentions that the hood portion allows the physician to perform suctioning of waste or the like during a resection or biopsy procedure, it teaches that the hood is specifically designed to avoid the suctioning of the mucous tissue. It may also be noted that the document does not mention the possibility of using such a hood with a clip device.

### SUMMARY OF THE INVENTION

The present invention relates to medical devices for grasping tissue using an endoscope flexible distal insert portion and for improving healthcare to individuals undergoing endoscopic procedures.

Aspects of the invention are set out in the appended claims.

The following disclosure describes a clip device delivery apparatus for causing homeostasis to a living tissue in a body cavity that comprises a haemostatic clip device and a protective cap. The haemostatic clip is configured to be detachably operatively coupled to an elongate deployment device, the clip device comprising a plurality of arms movable between a radially expanded position and a closed position. The protective cap has a proximal tube mounting section and a distal section, the distal section being configured to receive the clip arms in their radially expanded position.

The following disclosure also describes a method in which a clip device delivery apparatus is assembled. An elongate introducer tube having a working channel and distal end portion is provided. A clip device having clip arms is provided. The clip device is detachably operatively coupled to an operating wire that is inserted into the introducer tube working channel. A protective cap having a proximal tube mounting section and a distal section and a passageway therebetween is provided, the mounting section detachably secured to the tube distal end portion.

Furthermore, the following disclosure describes a system for delivering a clip device that has an endoscope, protective cap, and clip delivery system. The endoscope has a working channel and distal end portion. The haemostatic clip. is configured to be detachably operatively coupled to an elongate deployment device, the clip device comprising a plurality of arms movable between a radially expanded position and a closed position. The protective cap has a proximal tube mounting section and a distal section, the distal section being configured to receive the clip arms in their radially outward state.

### BRIEF DESCRIPTION OF THE DRAWINGS

Embodiments of the present invention will now be described by way of example only, and not by way of limitation, with reference to the accompanying drawings briefly described as follows:
Figure 1 is a schematic illustration, broken away, of an endoscope for use with the present invention.
Figure 2 shows an assembly that forms part of a clip delivery device according to an embodiment of the present invention.
Figure 3 provides a longitudinal, sectional view of a portion of a clip delivery device that forms part of a clip device delivery apparatus according to one embodiment of the present invention.
Figure 4 provides a longitudinal, sectional view of a detachable clip that forms part of clip deliver delivery apparatus according to an embodiment of the present invention.
Figure 5 is a longitudinal, sectional view of a protective cap that forms part of a clip device delivery apparatus according to one embodiment of the present invention.
Figure 6 is a longitudinal, sectional view of an embodiment of a protective cap secured to an endoscope and receiving a detachable clip device, thus providing a clip device delivery apparatus according to an embodiment of the present invention.
Figure 7 is a block diagram illustrating a method of grasping tissue endoscopically according to the present invention.

### DESCRIPTION OF THE INVENTION

Although not limited in its scope or applicability, the present invention relates generally to devices for grasping tissue endoscopically. More particularly, the invention relates to a clip and protective cap device for use with an endoscope, to grasp tissue endoscopically for performing hemostasis, tissue marking, endoscopic mucosal resection, tissue ligation, and a number of other applications related to colorectal medical procedures or gastrointestinal bleeding and the like.

For the purpose of promoting an understanding of the principles of the invention, the following provides a detailed description of embodiments of the invention as illustrated by the drawings as well as the language used herein to describe various aspects of the invention. The description is not intended to limit the invention in any manner, but rather serves to enable those skilled in the art to make and use the invention. As used herein, the terms comprise(s), include(s), having, has, with, contain(s) and variants thereof are intended to be open ended transitional phrases, terms, or words that do not preclude the possibility of additional steps or structure.

The novel features of the present invention of a clip device delivery apparatus with a protective cap will become apparent to those of skill in the art upon examination of the following detailed description of the invention or can be learned by practicing the present invention. It should be understood, however, that the detailed description of the invention and the specific examples presented, while indicating certain embodiments of the present invention, are provided for illustration purposes only, because various changes and modifications within the scope of the
invention will become apparent to those of skill in the art from the detailed description, figures, and claims that follow.

### PROVIDING AN ENDOSCOPE

According to the present invention there is provided a clip device delivery apparatus for a gastrointestinal endoscope.

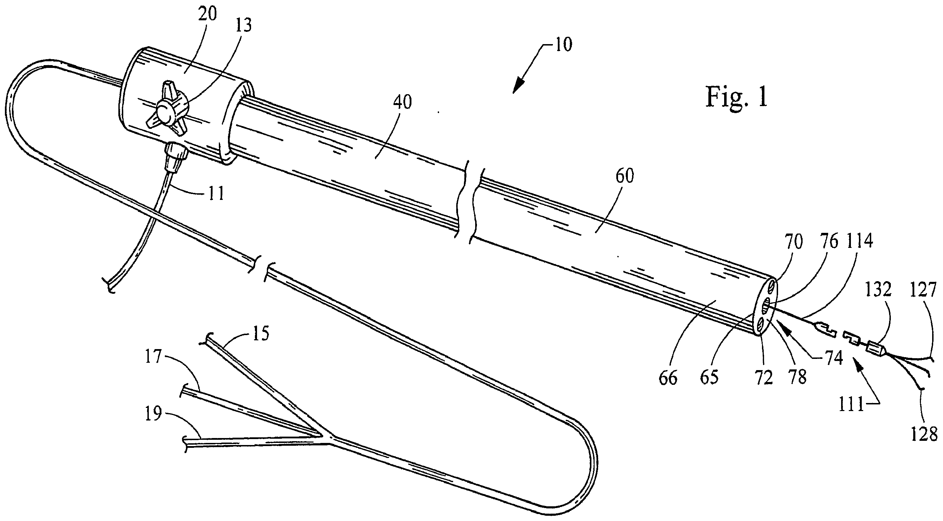

Figure 1 is a schematic illustration, broken away, of an endoscope 10 that is suitable for use with (or that may form a part of) a clip device delivery apparatus according to embodiments of the invention.. By way of background, endoscopic surgery has seen rapid growth over the past decade. A wide range of applications have been developed for the general field of endoscopes. Several applications include, by way of example only, some endoscopes that are rigid and other endoscopes that are flexible: arthroscope, angioscope, bronchoscope, choledochoscope, colonoscope, cytoscope, duodenoscope, enteroscope, esophagogastroduodenoscope (gastroscope), laparoscope, laryngoscope, nasopharyngoneproscope, sigmoidoscope, thoracoscope, and utererscope (individually and collectively, "endoscope").

As shown in Figure 1, a clip device delivery apparatus may comprise a conventional endoscope 10 having a proximal control section 20 and, extending distally therefrom, an elongate (long) introducer tube 40 (also referred to as an endoscope elongate introducer tube 40) having a distal insertion section 60. The introducer tube 40 and/or distal insertion section 60 optionally are flexible. The term "endoscope insert," "endoscope distal insert section," "distal insert section,"^{.} "endoscope distal insert," or "insert" shall include the distal insertion section 60 to be inserted into a patient, as well as any medical device such as a sheath, for example, that might be mounted onto the outer surface 66 of the distal end portion 65 of the distal insertion section 60. In other words, the outer surface 66 of the distal insertion section 60 includes the outer surface of a sheath that has been mounted onto the endoscope distal insertion section 60. As is conventional, the term "distal" means away from the gastroenterologist when the device is inserted into a patient, while the term "proximal" means closest to or toward the gastroenterologist when the device is inserted into a patient.

The proximal control section 20 remains outside the patient during a medical procedure and has several common features. One such feature includes an
eyepiece 15 for viewing the scene through a viewing lens 70 disposed at the distal insertion section 60. The viewing lens 70 may be a lens image sensor or any suitable means for viewing the scene through a viewing lens disposed at the distal insertion section 60. Other common features include an inlet 17 for passing a tool, a light delivery apparatus and a power supply 11, and an air or liquid supply and/or suction port 19 - which components ought to be understood by one of skill in the art. Endoscopes and systems are available from United States manufacturer, ACMI, and from Japanese imaging companies Fujinon, Pentax, Olympus, and Machida, with Pentax and Olympus the most notable suppliers of endoscopes and systems. Another feature may be one or more (often a pair) of articulation control knobs 13 for manipulating (bending and articulating) the position of the flexible distal insertion section 60.

As shown in Figure 1, the endoscope 10 comprises the distal insertion section 60 that comprises a light source 72 and a viewing lens 70 for visualizing the interior of an internal region of a body. In order to form an image of the scene under observation, the light source 72 and viewing lens 70 are located at or near a distal end portion 65 of the distal insertion section 60 to be inserted into a body cavity of a patient. Nowadays the light source 72 is outside the body and the light is typically directed via an optical fiber system to the distal end portion 65 of the distal insertion section 60.

The overall length and diameter of the elongate introducer tube 40 and distal insertion section 60 of the endoscope 10 may vary depending on the intended application for the endoscope. For instance, a standard colonoscope for insertion into the colon and distal terminal ileum typically measures approximately from approximately 1,330 millimeters ("mm") to approximately 1,850 mm in length and from about 11.1 mm to about 19 mm in diameter. The esophagogastroduodenoscope (the "gastroscope") used for insertion into the esophagus, stomach, and duodenum may have an insertion tube with a working length that measures approximately a meter, from about 925 mm to about 1,100 mm in length, and an insertion tube diameter from approximately 5.1 mm to about 12.8 mm. An example of a longer type of
endoscope is the enteroscope for insertion into the duodenum and proximal portion of the jejunum. The enteroscope may have an insertion tube that measures over 2 meters in length, from about 2,180 mm to about 2,800 mm, and an insertion tube diameter from approximately 5 mm to about 11.7 mm. A standard duodenoscope for endoscopic retrograde cholangio-pancreatography typically includes an insertion tube from about 1,030 mm to about 1,250 mm in length and from approximately 7.4 to approximately 12.6 mm in diameter. A standard choledoschoscope for passing through the channel of a duodenoscope or inserting intraductally for the bile and pancreatic ducts has an insertion tube length from about 1,870 mm to about 1,900 mm and an insertion tube diameter from approximately 2.8 mm to approximately 3.4 mm. An echoendoscope for the luminal digestive tract and adjacent organs may have an insertion tube length from about 975 mm to about 1,325 mm and an insertion tube diameter from approximately 7.9 mm to approximately 13.7 mm. An example of a shorter type of endoscope is the sigmoidoscope for the rectum and sigmoid colon. The sigmoidoscope may include an insertion tube that measures from about 630 mm to about 790 mm in length and a diameter from approximately 12.2 mm to approximately 13.3 mm.

Endoscopes may also incorporate additional functionality for operation within the body, such as a working channel 74 having a distal opening 76 located at the distal end portion 65 of the distal insertion section 60 of the endoscope 10. In one embodiment, the distal end portion 65 is located at a distal end face 78 of the distal insertion section 60. Similar to the variable lengths and diameters of the different types of endoscopes, the working channels vary in diameter: gastroscope (≈2.0-6.0 mm); enteroscope (≈1.0-3.5 mm); duodenoscope (≈2.0-4.8 mm); choledochoscope (≈0.75-1.2 mm); echoendoscope (≈2.2-3.7 mm); colonoscope (≈2.8-4.2 mm); and sigmoidoscope (≈3.2-4.2 mm).

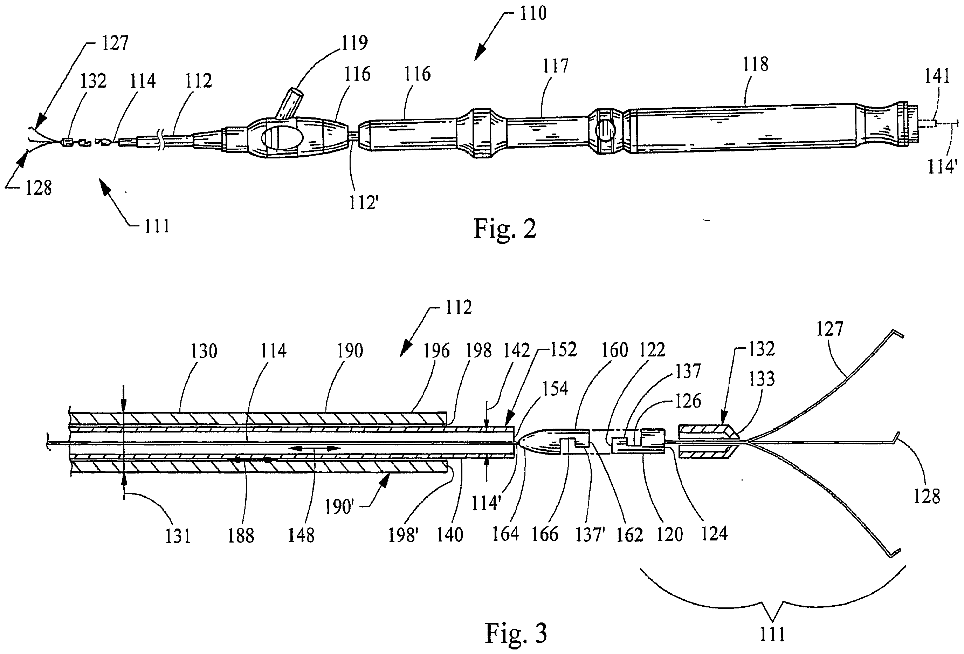

The term "working channel" as used herein and throughout this description is understood to be any passageway, lumen, chamber, channel, opening, bore,
orifice, flow passage, duct, or cavity that is sized, configured, and/or capable of slideably receive and allow the conveyance, passage, or movement of another component of the clip device delivery apparatus. For instance, the working channel 74 may slideably receive and allow an elongate deployment device wire 114 (Figs. 2-3), an inner sheath 140 (Fig. 3), and/or an elongate introducer tube 130 (Fig. 3). In an embodiment wherein the clip device delivery apparatus (See Figs. 2-3 discussed below) includes an assembly 110 that comprises an elongate introducer tube 112 that is an outer sheath, then the sheath may comprise a working channel 188 for slideably receiving an inner sheath 140, which inner sheath may comprise a working channel 148 for slideably receiving an elongate deployment device 114. In one embodiment, the elongate deployment device 114 is an elongate operating wire. An elongate deployment device 114 may be referred to as an elongate operating wire 114 hereafter, but it should be understood that an elongate operating wire 114 in describing embodiments of the invention may be any deployment device that detachable operatively couples the clip device 111 and/or any portion or feature of the clip device 111. For simplicity, future reference to an elongate deployment device 114 will be to an elongate operating wire 114.

Through the working channel 74 of the endoscope 10, the gastroenterologist may apply suction or provide saline solution via the port 19 and directed to the distal opening 76 at the distal end portion 65 of the distal insertion section 60. Also, the gastroenterologist may pass various types of diagnostic, monitoring, treating, or surgical tools through the working channel 74 via the inlet 17 and out the distal opening 76 to a site external to the distal end face 78 and into the observation field and working space of the gastroenterologist' s endoscope 10. For instance, the gastroenterologist may introduce endoscopic clipping devices through the endoscope working channel 74 and out the opening 76 at the distal end portion 65. In one embodiment, the distal opening 76 is located at the distal end face 78 of the distal insertion section 60.

The clip device delivery apparatus 10 may further comprise an elongate (long) operating wire 114 and a dip device 111, wherein the clip device 111 is
configured to grasp tissue endoscopically for performing hemostasis, tissue marking, endoscopic mucosal resection, tissue ligation, and a number of other applications related to colorectal medical procedures or gastrointestinal bleeding and the like. The clip device 111 comprises a plurality of clip arms 127, an optional tooth 128 on each clip arm or on a plurality of clip arms, and a sliding ring 132 configured to slide over the clip arms. The clip device 111, operating wire 114, and other features of embodiments of the clip device delivery apparatus are described below in greater detail in connection with the clip device delivery apparatus 110 of Figures 2 and 3. Also, a clip device is described in relevant part in United States Patent No. 7,122,041 B1 entitled, "Clip Device", and a multi-clip device is described in relevant part in Published United States Patent Application 2006/0224165 (Surti et al.) entitled, "Multi-clip Device".

### PROVIDING A CLIP DEVICE

Figure 2 shows an assembly 110 that forms a part of a clip device delivery apparatus according to another embodiment of the invention. The assembly 110 for the clip device delivery apparatus may be used with an endoscope 10. The assembly 110 for a clip device delivery apparatus includes an elongate (long) operating wire 114 (also referred to as a drive cable) disposable - and independently slideable - within an optional elongate introducer tube 112 that is an outer sheath (also referred to as an outer sheath elongate introducer tube 112). In other words, the operating wire 114 can be advanced and retracted independently of the movement of the introducer tube 112. The introducer tube 112 is optional in the event that the gastroenterologist may use the endoscope working channel 74 and its interior tubular wall (see Fig. 1) in place of and functioning as the introducer tube 112 for constraining and positioning a clip device 111 comprising a sliding ring 132 and plurality of arms 127 that may optionally comprise an inwardly facing tooth (or bend) 128.

The overall length of the operating wire 114 and introducer tube 112 may vary depending on the intended medical procedure and, if used with an endoscope, depending on the length of the endoscope and its working channel 74. For instance, if used with an endoscope, the lengths of the operating wire 114 and introducer tube 112 generally correspond approximately to the length of the endoscope working channel 74 and/or at least the length of the distal insertion section 60 to be inserted into a patient. Likewise, the overall outer diameter of the introducer tube 112 may vary depending on the inner diameter of the endoscope working channel 74 to be used with, and may taper (reduced cross section) in the distal direction, but generally is less than the inner diameter of the working channel 74 in order to be slideable therein. During use, a gastroenterologist can advance and retract the operating wire 114 independently of the introducer tube 112.

The introducer tube 112 optionally attaches at its proximal end 112' to handle assembly 117 that comprises a forward handle portion 116 and a rearward handle portion 118 that is proximal relative to the forward handle portion 116. In one embodiment, the forward handle portion 116 optionally operatively couples to the proximal end 112' of the introducer tube 112. The operating wire 114 extends through the forward handle portion 116 and attaches at its proximal end 114' to the rearward handle portion 118, which rests proximally of the forward handle portion 116. In the embodiment wherein the assembly 110 for the clip device delivery apparatus comprises an introducer tube 112 further comprising an outer sheath 130 and an inner sheath 140, the inner sheath proxima1 end 141 may also be attached to the rearward handle portion 118, and may be operatively coupled to the operating wire proximal end 114'. The inner sheath proximal end 141 and operating wire proximal end 114' are shown in phantom to denote that they may be operatively coupled to the rearward handle portion 118 anywhere along the length of (and within) the rearward handle portion 118, or may be operatively coupled to (but extending proximal of) the rearward handle portion 118. The rearward handle portion 118 telescopically extends over the proximal portion of the forward handle portion 116. As will be explained in more detail below, the gastroenterologist
controls the longitudinal movement of the operating wire 114 relative to the introducer tube 112 by manipulating the forward handle portion 116 relative to the rearward handle portion 118.

By way of example only and not by way of limitation, the terms "operatively couple," "operatively coupling," "operatively coupled," "coupling," "coupled," and variants thereof are not used lexicographically but instead are used to describe embodiments of the invention having a point, position, region, section, area, volume, or configuration at which two or more things are mechanically, chemically, and/or chemical-mechanically bonded, joined, adjoined, connected, associated, united, mated, interlocked, conjoined, fastened, held together, clamped, crimped, friction fit, pinched, press fit tight, nested, wedged, and/or otherwise associated by a joint, a junction, a juncture, a seam, a union, a socket, a melt bond, glue, adhesives, resins, welding (laser, spot, etc.), soldering, brazing, adhesives, chemical bonding materials, implanted arrangement, or combinations thereof. Embodiments of latching members include latches, screws, clamps, cams, hooks, sleeves, collets, and the like.

The forward handle portion 116 also includes an optional flushing port 119 comprising a standard male or female luer fitting or any other valve mechanism that permits the injection of fluid there through. The flushing port 119 is in fluid communication with the interior volume of the forward handle portion 116, which in turn is in fluid communication with the working channel 188 within the introducer tube 112. Accordingly, any fluid injected through the flushing port 119 will necessarily enter the working channel 188 of the introducer tube 112, and will exit the cavity near the distal opening 198' of the introducer tube 112 (see Fig. 3). In other words, the fluid injected through the flushing port 119 will exit a distal end portion 190' (see Fig. 3) of the assembly 110 forming part of the clip device delivery apparatus.

The term "fluid communication" and variants thereof are not used lexicographically but instead are used to describe embodiments of the invention such that, by way of example only and not by way of limitation, the flushing port 119 and the working channel 188 (see Figs. 2 and 3) are configured to allow conveyance, regulation, flow, and/or movement of fluids, medication, and/or gases
there through. Alternatively, the flushing port 119 and the working channel 188 can be separated by additional intervening spacings, passageways, and/or occlusions, bushings, diaphragm seals, and/or valves (and/or valve/diaphragm seal equivalents) configured to open/close and otherwise allow fluid communication of the flushing port 119 and the working channel 188.

Alternatively, a lumen can be disposed within the wall of the introducer tube 112. In other words, the introducer tube can comprise a separate lumen disposed through which fluid can be passed along the length thereof. Optionally, the assembly 110 for the clip device delivery apparatus uses a working channel 148 comprising an inner diameter 142 in an inner sheath 140 (the inner sheath 140 is discussed below and illustrated in Figure 3). As yet another alternative, it should also be understood that the flushing port 119 could be located on the middle section of the rearward handle portion 118, or on a section of the introducer tube 112 distally of the forward and rearward handle portions 116, 118, respectively.

As shown in Figures 2 and 3, the assembly 110 for the clip device delivery apparatus includes at least one detachable clip device 111 disposable within the introducer tube 112. The clip device 111 extends distally from the elongate operating wire 114 and is removably attached to the elongate operating wire 114. Thus, the "clip" device is not forceps (or similar device) but is detachable to the elongate operating wire 114. In another embodiment according to the invention, the clip device delivery apparatus is capable of delivering a plurality (e.g., two or more) of detachable clips arranged inside the introducer tube in a serial fashion. By way of example only and not by way of limitation, the distal end of the operating wire engages the engagement portion of the proximal most clip device, and the arms of the proximal most clip device engage the engagement portion of the adjacent distally located clip device. Additional clip devices, if included, are connected to each other in the same "head-to-tail" fashion. In order words, each clip device is connected to the operating wire via each of the proximally located intervening clip devices.

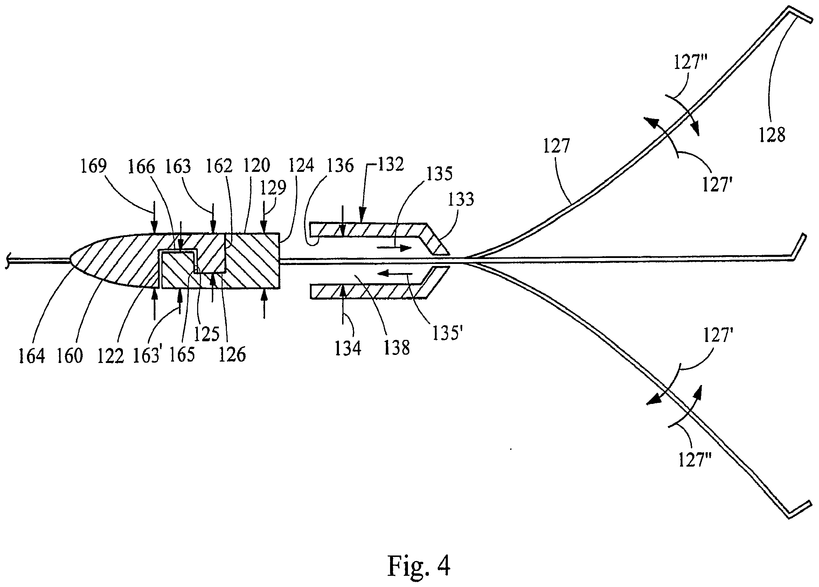

As illustrated in Figures 3 and 4, each clip device 111 has a proximal portion 14 and distally has a plurality of arms 127. The arms 127 are formed of a resilient material and are shaped such that the arms 127 have a self-expanding tendency such that the clip arm distal teeth are spaced apart from each other when the clip arms are in an open position 127' and substantially adjacent when the clip arms are in a closed position 127" (e.g., Fig. 4). Optionally, one or more arms 127 includes an inwardly facing tooth (or bend) 128 configured to grasp the target tissue. The tooth 128 of one arm 127 may overlap with another arm or the tooth of another arm, when the clip device 111 is in the closed position. As will be explained in greater detail below, a second end 122 comprising a notch 126 configured to operatively couple a second retainer 160, or the closed arms 127 of another clip device 111 may engage.

In the embodiment illustrated in Figure 3, the clip device 111 comprises a plurality of arms 127. The arms 127 (as shown by example only) each have a tooth 128, but not every arm according to the invention has to have a tooth. The tooth 128 may be formed by bending a single elongate piece of resilient material.

In the embodiment illustrated in Figure 4, the clip device 111 comprises three arms 127, but three arms are not necessarily important to the clip device 111, which could also work with two arms. Each arm 127 is individually formed from a resilient material and affixed to a proximal portion 14 that comprises a first retainer 120 (discussed below) by any suitable means such as welding, gluing, crimping, or other mechanical attachment. The use of two arms (or in one embodiment three arms) allows the clip device 111 to grasp the target tissue with minimal, if any, need to rotate the clip device 111 into the correct orientation. While three arms are illustrated in this embodiment, it is contemplated that more than three arms may be used in other embodiments.

The clip device 111 may be made from any suitable resilient material such as stainless steel, nickel-titanium alloy ("nitinol"), plastic, and the like, and is preferably a biocompatible material. The material used for the clip device 111 may also be bio-degradable. In addition, the arms 127 may have a cross-sectional
shape that is round, square, triangular, pie-shaped, truncated cone, and the like. A triangular or delta shaped cross-section is particularly advantageous for a clip device having three arms because it allows a reduction in the cross-sectional area that the arms occupy within the working channel 74, 148, 188 of an introducer tube 40, 112, thereby allowing a reduced diameter for the introducer tube 40, 112.

Figure 3 illustrates an introducer tube 112 comprising an outer sheath 130 having a distal insertion section 190 comprising a distal end portion 190' comprising an outer surface 196 and a distal opening 198 optionally at a distal end face 198'. The outer sheath 130 further comprises a working channel inner diameter 131 sized to slideably receive an inner sheath 140. The gastroenterologist may advance the inner sheath 140 and retract the inner sheath 140 independently relative to the outer sheath 130. The inner sheath 140 has a distal end portion 152, a distal opening 154, and a working channel inner diameter 142 that slideably receives the operating wire 114. The inner sheath distal end portion 152 may extend distally, may retract proximally, or may align substantially co-planar relative to the distal insertion section 190 of the outer sheath 130. Furthermore, the working channel inner diameter 131 is greater than an outer diameter of the inner sheath 140 for facilitating the slideable engagement of the outer and inner sheaths 130, 140, respectively, and/or for passing fluids directly or indirectly from the flushing port and to exit subsequently at or near the outer sheath distal insertion section 190. In addition, the inner sheath 140 is configured to slideably receive an assembly comprising first and second retainers 120, 160, respectively, of the clip device 111.

Figures 3 and 4 show the illustrative embodiments, in which the first and second retainers 120, 160, respectively, of the clip device 111 are in two configurations. Figure 3 shows the first retainer 120 and second retainer 160 spaced apart (detached), while Figure 4 shows the retainers 120, 160 joined detachably together.

In one embodiment, the second retainer 160 attaches to the distal end 114" of the operating wire 114. The first
and second retainers 120, 160, respectively, are configured to detachably join together. In one illustrative embodiment, the first retainer 120 has a first end 124 and a second end 122 comprising a notch 126 proximal to the first end 124, the notch 126 optionally configured to engage a hook 137' of the second retainer 160 (compare Figures 3 and 4). The first retainer 120 has a shape that will complement a shape corresponding to the second retainer 160 so that the first and second retainers can detachably join together. For example, the first retainer hook 137 may have a semi-circular cross-section and the first retainer notch 126 has a semi-circular cross-section, the cross sectional area of the hook 137 being greater than that of the notch 126 (compare Figure 3 showing perspective schematic views of the first and second retainers 120, 160, respectively, with Figure 4 showing a longitudinal sectional view of same). Accordingly, in one illustrative embodiment the second retainer 160 has a first end portion 162 and a second end portion 164 with a notch 166 disposed between the first end portion 162 and the second end portion 164. By way of example only and not by way of limitation, in one embodiment the second retainer 160 at or near the first end portion 162 has a first diameter 163 and, at the second end portion 164, the second retainer 160 is in the shape of a half-cylinder having an engaging surface 165. In addition, the first diameter 163 of the second retainer 160 is substantially similar to a first diameter 163' of the first retainer 120.

In one illustrative embodiment, the first and second retainers 120, 160, respectively, detachably join to each by locating an engaging surface 125 of the first retainer within the second retainer notch 166 and by locating the second retainer engaging surface 165 within the first retainer notch 126. Optionally, the first retainer second end 122 and the second retainer first end portion 162 are approximately one-half the diameter of the first diameters 163', 163 of their respective retainers, when joined, the first and second retainers form a substantially continuous cylinder shape having substantially the same diameter, but the retainers may taper such as the second retainer second end portion 164 shown in Figures 3 and 4.

A sliding ring 132 will now be discussed in further detail, as shown in Figures 3 and 4. It will be understood by one of skill in the art that, although the first and second retainers detachably join with each other, they will not retain a joined position unless they are held together. Accordingly, there is a sliding ring 132. The sliding ring has an inner diameter 134 slightly larger than an outer diameter 129 the first retainer 120 but slightly less than an outer diameter 169 of the second retainer 160 proximal the second retainer notch 166. In other words, the inner diameter 134 of the sliding ring 132 is such that the sliding ring 132 can slide over the notches 126, 166 wherein the retainers are operatively coupled at the notches 126, 166 so as to hold and maintain the retainers 120, 160 in a detachably joined position. As a result, the sliding ring 132 can slide over the first and second retainers 120, 160 to hold them in a mating position, and the inner diameter 142 of the inner sheath distal end portion 152 is large enough to slide over the second retainer outer diameter 169 and first retainer outer diameter 139 but is less than the sliding ring inner diameter 134 so that the inner sheath distal end portion 152 can be used to push the sliding ring 132 distally off the retainers 120, 160. Alternatively, the inner diameter 134 may be smaller than the first diameter 163' of the first retainer 120 (e.g., Fig. 4). In such an embodiment, the retainers 120, 160 would be held together by the distal end portion 152 (Fig. 3) of the inner sheath 140.

Figures 3 and 4 show that a distal end portion 133 of the sliding ring 132 preferably has an inner diameter 134 (i.e., lumen 138) smaller than the first diameter 163' on the first retainer 120. As a result, the sliding ring 132 is not removable from the clip device 111. The sliding ring 132 can abut the first retainer first end 124 so that the clip arms 127 are in an open position 127' (see Fig. 4). The sliding ring 132 can then be moved distally over the arms 127 to close them into an engaging closed position 127" (see Fig. 4).

The sliding ring 132 further comprises a lumen 138 sized for slideably receiving and closing the clip arms 127 as the sliding ring 132 advances distally over the clip device 111 as discussed below. In other words, the wall portion of the sliding ring 132 that defines the lumen 138 is configured to engage and overcome the transverse outwardly directed biasing force of the arms 127 so as to push the teeth 128 of the clip arms 127 into an overlapping arrangement as the sliding ring 132 moves distally over the clip arms 127. The lumen 138 may also be sized so as to prevent the first end 124 of the first retainer 120 from passing there through and thereby preventing the sliding ring 132, such that the sliding ring 132 is retained proximally by the first retainer 120 and retained distally by the engaged clip arms 127 and/or engaged teeth 128 of the clip arms 127.

Figure 4 shows that the sliding ring 132 is movable between a sliding ring first position 135 disposed about the plurality of clip arms 127 to move the clip arms to a closed position 127" and a sliding ring second position 135' that allows the clip arms to be spaced apart from each other in an open position 127'. Optionally, the first retainer distal end 124 is disposed within the sliding ring lumen 138 when the sliding ring 132 is in the sliding ring second position 135' and is disposed proximally of the sliding ring 132 when the sliding ring 132 is in the sliding ring first position 135. Optionally, the first retainer 120 has a circular cross-section defining an outer diameter 129, the second retainer has a circular cross-section defining an outer diameter 169, and the sliding ring inner diameter 134 is slightly larger than the first retainer outer diameter 129 but slightly less than the second retainer outer diameter 169. Optionally, the sliding ring 132 has an interior surface 136 that is approximately equal to that of the first retainer outer diameter 129. Optionally, the sliding ring 132 has distal portion 133 that is tapered such that the distal portion 133 is smaller than the first retainer outer diameter 129 in order to prevent the sliding ring from sliding proximally off of the first retainer 120.

The exterior surface and outer diameter of the sliding ring 132 may also be sized and/or configured to fit loosely within the introducer tube 112 in an arrangement that allows the passage of fluids around or through the sliding ring 132. As will be explained below, it may be desirable, for example, to pass saline through the introducer tube 112 to flush any blood or bodily fluids away from the part to be treated. Thus, the sliding ring 132 may be sized to provide a gap between the exterior sliding ring surface and the interior surface of the introducing tube 112 through which fluids can pass. Alternatively, the sliding ring 132 may include a flow channel or lumen extending there through.

The sliding ring 132 may be made from any suitable resilient material such as plastic, rubber, stainless steel, nitinol, and the like, and is preferably a biocompatible material. The material used for the sliding ring 132 may also be bio-degradable. The sliding ring 132 can be manufactured by any suitable procedure, such as milling (in the case of metal materials) or injection molding (in the case of plastic and rubber materials).

Certain details of the operation of one embodiment will be described. An assembly 110 forming part of a clip device delivery apparatus 110 is provided with an outer sheath 130 that is retracted to expose the inner sheath 140, the operating wire distal end 114", and a clip device 111 comprising a first retainer 120 detachably joined with a second retainer 160. Optionally, the sliding ring 132 may be pushed over the first and second retainers 120, 160, respectively, so that the retainers are maintained in a joined position.

The outer sheath 130 is pushed toward the distal end 152 of the inner sheath 140 and beyond the clip device 111 causing the arms 127 to close. In this state, a gastroenterologist provides an endoscope and inserts a distal insertion section 60 of the endoscope into the body cavity, and then introduces the introducer tube 112 into the body cavity via the endoscope working channel 74. While observing the body cavity via the endoscope, the gastroenterologist guides the outer sheath distal end portion 190 to a target site.

When by blood or other bodily fluids at the target site obscures the endoscope's observation field, and then the gastroenterologist may inject a fluid such as saline through the flushing port 119 on the forward handle portion 116. The saline enters the working channel 188 of the introducer tube 112 between the inner and outer sheaths 140, 130, respectively, and exits the distal opening 198 of the distal end portion 190' of the outer sheath 130. The saline floods the area so as to flush any blood or bodily fluids away from the part to be treated. Saline may be injected or repeated as necessary during the following steps so as to keep the area free of blood and other bodily fluids.

Alternatively, a gastroenterologist may apply a vacuum to the flushing port 119 so as to create suction within the working channel 188 of the introducer tube 112 between the inner and outer sheaths 140, 130. This suction can remove blood or other bodily fluids from the area surrounding the part to be treated.

Next, pulling the outer sheath 130 proximally (i.e., retracting the outer sheath) exposes the clip device 111 and the inner sheath distal end portion 152. Advancing the inner sheath 140 toward the clip device 111 causes the sliding ring 132 to slide distally toward the clip arms 127, thereby causing the arms 127 to close about the target tissue. The inner sheath 140 is then retracted and when the distal end of the inner sheath passes the first and second retainers, they detach and release from each other because the sliding ring 132 has slid distally over the clip arms 127 and, therefore, the sliding ring no longer holds the retainers together. The clip remains inside the body cavity and holds the tissue. After disengaging the retainers as described, the gastroenterologist removes the clip operating device 110 from the endoscope working channel 74.

In the second embodiment according to the clip device delivery apparatus 110, the gastroenterologist retracts the outer sheath 130 to expose the inner sheath 140, the operating wire distal end 114", and the second retainer 160. A clip device 111 according to this embodiment of the present invention has a first retainer 120 that detachably joins with the second retainer 160, and the sliding ring 132 maintains the retainers 120, 160 in a joined position.

Next, the gastroenterologist pushes the outer sheath 130 toward the inner sheath distal end portion 152 and beyond to the clip device 111, which causes the clip arms 127 to close. In this state, a gastroenterologist provides an endoscope 10 and inserts its distal insertion section 60 into the body cavity, and then advances the introducer tube 112 into the body cavity via the endoscope working channel 74. While observing the body cavity via the endoscope, the gastroenterologist guides the outer sheath distal insertion section 190 to a target site.

Then, pulling the outer sheath 130 proximally exposes the clip device 111 and the inner sheath distal end portion 152. Advancing the inner sheath 140 toward the clip device 111 moves the sliding ring 132 over the clip arms 127 and
causes the arms 127 and teeth 128 to close about the tissue. The inner sheath 140 is then retracted and when the inner sheath distal end portion 152 passes the first and second retainers 120, 160, they detach and release from each other because the sliding ring 132 has slid distally over the clip arms 127 and, therefore, the sliding ring 132 no longer holds the retainers 120, 160 together. The clip device 111 is left inside the body cavity, holding the tissue. After disengaging the retainers as described, the gastroenterologist removes the clip device delivery apparatus 110 from the endoscope working channel 74.

In yet another embodiment, the outer sheath 130 is omitted. The working channel 74 slideably receives the operating wire 114 detachably secured to the clip device 111, wherein the inner sheath 140 receives the operating wire 114 but the clip device extends distal of the inner sheath distal end portion. Advancing the inner sheath distal end portion 152 toward the clip device 111 moves the sliding ring 132 over the clip arms 127 and causes the arms 127 and teeth 128 to close about the tissue. The inner sheath 140 is then retracted and when the inner sheath distal end portion 152 passes the first and second retainers 120, 160, they detach and release from each other because the sliding ring 132 has slid distally over the clip arms 127 and, therefore, the sliding ring 132 no longer holds the retainers 120, 160 together. The clip device 111 is left inside the body cavity, holding the tissue. After disengaging the retainers as described, the gastroenterologist removes the clip device delivery apparatus 110 from the endoscope working channel 74.

It should be understood that a multi-clip device also may be provided, whereby one or more clip devices 111 can be initially loaded into the introducing tube 112. Each clip device 111 would then be available for deployment during the medical procedure without the need to withdraw the clip device delivery apparatus for re-loading after each deployment, and without the need to insert additional clip delivery devices. Materials and methods of manufacturing and using a multi-clip device are described in Published United States Patent Application 2006/0224165 (Surti et al.) entitled, "Multi-clip Device".

While there have been described what are presently believed to be the preferred embodiments of clip device delivery apparatus 10, 110 according to the invention, those skilled in the art will realize that changes and modifications may be made thereto without departing from the invention. It is to be understood that the invention can be carried out by specifically different equipment and devices, and that modifications, both as to the equipment and operating procedures, can be accomplished without departing from the scope of the invention itself.

### PROVIDING A PROTECTIVE CAP

According to the present invention, the clip device delivery apparatus comprises a detachable protective cap that comprises a proximal tube mounting section that detachably fits over a distal end portion of a gastrointestinal endoscope.
In one embodiment, the clip device delivery apparatus comprises a protective cap 210 attached to the outer surface 66 of a distal end portion 65 of an endoscope distal insertion section 60. The protective cap 210 will now be described in detail.

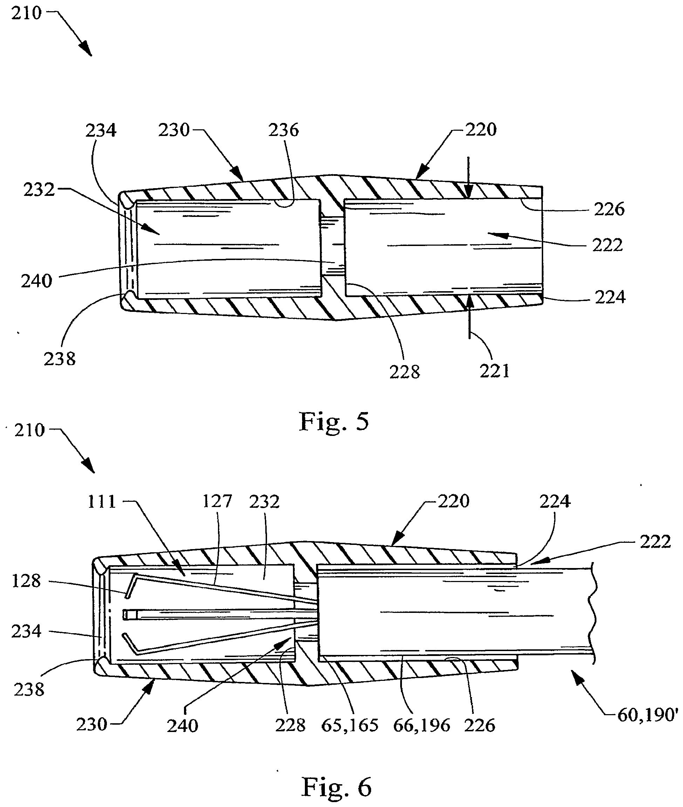

Figure 5 shows a sectional side view of a protective cap 210 that forms a part of a clip device delivery apparatus according to one representative embodiment of the present invention. A protective cap has a proximal tube mounting section 220 and a distal section 230, both of which are approximately tubular.

The tube mounting section 220 has a tubular configuration and comprises a tube receiving lumen 222. In describing embodiments of the invention, the term "tube" and "tubular" shall have their plain and ordinary meaning and include any shaft-like, rounded, oblong, circular, tube-like, tubular, or cylindrical structure having a lumen. The protective cap can comprise other shapes, and in one embodiment comprises a bevel or beveled shape. A proximal opening 224 allows the tube receiving lumen 222 to receive the a distal end portion 65 of a distal insertion section 60 A tube engaging inner surface 226 detachably mounts the protective cap 210 onto the outer surface 66 of the distal end portion 60 .

The opening 224, lumen 222, and engaging surface 226 are sized to snugly but detachably secure the outer surface 66 in a compression fit that (optionally) substantially forms a hermetic seal until the protective cap 210 is removed from distal end portion 60. One or more tube stops 228 protrude inwardly toward the tube receiving lumen 222 to abut and/or inhibit (e.g., limit, control, and/or stop, etc.) distal progression of a distal end face 78 of the distal insertion section 60, thereby prevent distal movement into the distal section 230. The tube stop 228 may comprise a flange, protrusion, or ridge of any geometric shape, dimension, or perimeter. In one embodiment, the tube stop 228 is a ringed structure that protrudes inwardly toward the tube receiving lumen 222. A passageway 240 extends between the stop(s) 228 so as not to occlude the endoscope working channel 74, 188, and/or 148, the opening 76, 198, and/or 154, the distal end portion 190' and/or 152, and/or the lens 70 and light 72 at the endoscope distal end face 78.

Because the mounting section 220 fits substantially concentrically over at least a portion of a distal end portion 65 of an endoscope distal insertion section 60, the mounting section 220 comprises an inner diameter 221 sized to accommodate the distal end portion 65. In one embodiment, the mounting section inner diameter 221 is at least about 5.0 mm up to about 19.0 mm, or may be greater or less than this range at certain positions along the length of the mounting section given any tapering in the inner diameter 221 in order to snugly fit to the outer surface 66 of the distal end portion 65. The length of the tube receiving lumen 222 of the mounting section 220 may vary, and in one embodiment the length is in a one-to-one ratio (e.g., at least about 5.0 mm up to about 19.0 mm) with the mounting section inner diameter 221, although the length may be greater than a one-to-one ration.

Endoscopes may also incorporate additional functionality for observation or operation within the body, such as a working channel having an opening located at the distal end portion of the insert. Similar to the variable lengths and diameters of the different types of endoscopes, the working channels vary in diameter: gastroscope (≈2.0-6.0 mm); enteroscope (≈1.0-3.5 mm); duodenoscope (≈2.0-4.8 mm); choledochoscope (≈0.75-1.2 mm); echoendoscope (≈2.2-3.7 mm); colonoscope (≈2.8-4.2 mm); and sigmoidoscope (≈3.2-4.2 mm).

According to the present invention, the distal section 230 is configured to receive tissue during a medical procedure, such as performing by way of example and not by way of limitation hemostasis, tissue marking, endoscopic mucosal resection, tissue ligation, and many other applications related to colorectal medical procedures or gastrointestinal bleeding and the like. The distal section 230 comprises clip arm receiving chamber 232, which is configured to receive the subject tissue to be grasped by arms 127 of the clip device 111. An inner surface 236 constrains the expanding clip arms 127, which helps to guide each respective clip arm 127 in its own plane as the clip arm advances distally toward, in one embodiment, the subject tissue. At least one optional flange 238 protrudes inwardly toward the clip arm receiving chamber 232. The flange 238 abuts the arm's tooth 128 and thereby prevents the arm's distal movement beyond a distal opening 234.

Figure 5 shows the clip arm receiving chamber 232 having an inner diameter approximately equal to the inner diameter of the tube receiving lumen
222 of the endoscope mounting section 220. However, the tissue receiving section may be any flared tubular, square, rectangular, conical, or otherwise structure configured to receive clip arms and/or tissue and having an effective inner diameter greater than that of the endoscope receiving lumen 222.

The protective cap 210 may be made of any suitable material (natural, synthetic, plastic, rubber, metal, composite, or combination thereof). In one embodiment, the protective cap 210 comprises a hard, clear plastic such as comprising a polycarbonate, especially when visibility may be important to performing an endoscopic procedure that utilizes the protective cap 210. In general, the material may comprise a synthetic material that may include, for example, polyurethane, cellulose acetate, cellulose nitrate, silicone, polyethylene teraphthalate, polyamide, polyether block amide, polyester, polyorthoester, polyanhydride, polyether sulfone, polycarbonate, polypropylene, high molecular weight polyethylene, polytetrafluoroethylene, or mixtures or copolymers thereof, polylactic acid, polyglycolic acid or copolymers thereof, a polyanhydride, polycaprolactone, polyhydroxybutyrate valerate, polyhydroxyalkanoate, or another polymer or suitable material. Further, the material may be biocompatible or capable of being made biocompatible, such as by coating, chemical treatment, or the like. In general, the distal section 230 may be substantially rigid, while the tube mounting section 220 is less rigid so as to stretch and conform to (and form an airtight engagement with) the distal end portion 65 of the distal insertion section 60 of a clip device delivery apparatus 10 and/or distal end portion 190' of a distal insertion section 190 of an outer sheath 130 when the clip device delivery apparatus 110 is used blindly without an endoscope.

Figure 6, in a sectional schematic side view, illustrates a protective cap 210 detachably secured to a distal insertion section 60, and further shows arms 127 of a clip device 111 extending into a clip arm receiving chamber 232 of the protective cap 210. The cap's proximal tube mounting section 220 has an opening 224 that receives the distal insertion section 60 such that the cap's tube engaging inner surface 226 detachably mounts the protective cap 210 onto the outer surface 66 of the distal end portion 60 when the clip device delivery
apparatus 10 comprises an endoscope or detachably mounts the protective cap 210 onto the outer surface 196 of the distal end portion 190' of an outer sheath 130 when the clip device delivery apparatus 110 used blindly without an endoscope. In order to show the outer surface 66, 196 and inner surface 226, Figure 6 shows those features spaced apart, but in use they would abut into an airtight engagement such as a friction fit. The distal end face 78, 198' abuts the tube stop 228. A passageway 240 allows mechanical and visual communication between the cap's tube receiving lumen 222 and the clip arm receiving chamber 232.

The clip arm receiving chamber 232 has an opening 234 for receiving target tissue. In one embodiment, suction through the endoscope suction port 19 communicates suction to the clip arm receiving chamber 232 through the working channel 74, 188 (see Figs 1-3), an optional suction port 119 (see Fig. 1), and another optional channel in the endoscope, outer sheath, or an endoscope accessory tool or device. As schematically represented in Figure 6, the clip arms 127 have expanded to their radially expanded position 127' (in one embodiment a biased transverse outward direction) until constrained transversely by the cap's inner surface 236 and inhibited (e.g., limited, controlled, and/or stopped, etc.) distally by an optional flange 234.

### METHODS OF USING A CLIP DEVICE, PROTECTIVE CAP, AND ENDOSCOPE TOGETHER FOR GRASPING TISSUE

The foregoing discussion provided several steps of assembling and using a clip device delivery apparatus that comprises an endoscope 10 and or outer and inner sheaths 130, 140, respectively, a clip device 111, and a protective cap 210.

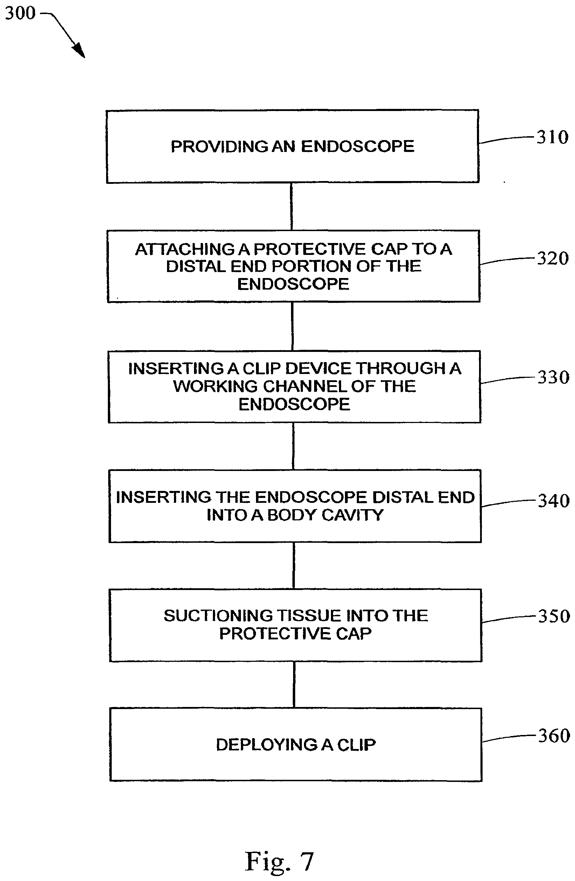

Figure 7 further shows an example of a method 300 for grasping tissue endoscopically to perform hemostasis using an assembly of the device described above and shown in Figures 1-5. This method 300 uses a protective cap
210 and a clip device delivery apparatus, which includes an assembly 110, with an endoscope (see Fig. 1 reference number 10, for grasping tissue comprises the steps of: providing an endoscope 10 (step 310); attaching a protective cap 210 onto the distal end portion 60 (e.g., outer surface 66) of the endoscope (step 320); inserting a clip device 111 through a working channel 74 of the endoscope (step 330); inserting the endoscope distal end portion 60 into a body cavity (step 340); suctioning tissue into the protective cap (step 350) such as into the clip arm receiving chamber 232; deploying a clip device 111 (step 360).

More particularly, the endoscope 10 that is provided (step 310) includes a working channel 74 and working channel distal opening 76, a lens 70 for viewing distally into a working area, and light 72 for illuminating the working area. The protective cap 210 that is provided (step 320) has a proximal tube mounting section 220 with a tube engaging inner surface 226 and a distal clip arm receiving chamber 232 with an inner surface 236 and at least a flange 234. At least one tube stop 228 and a passageway 240 intermediate the tube mounting section 220 and distal section 230 of the protective cap 210 is provided, the tube stop 228 providing controlled communication between the tube mounting section 220 and the clip arm receiving chamber 232 and configured so that the protective cap 210 does not occlude the endoscope working channel 74, distal opening 76, lens 70, or light 72 at the distal end face 78. The protective cap's proximal tube mounting section 220 is detachably secured onto an endoscope's outer surface 66 of a distal end portion 65 of an endoscope distal insertion section 60 until the endoscope distal end portion 65 abuts against the stop(s) 228, the cap inner surface 226 substantially forming a seal against the endoscope outer surface 65.

Moreover, the assembly 110 that forms part of the clip device delivery apparatus and that is inserted into the endoscope 10 (step 330) passes through the endoscope working channel 74, the assembly 110 having an elongate introducer tube 112 secured proximally to a forward handle 116, an elongate operating wire 114 secured proximally to a rearward handle 118 and being slideably disposed within the introducer tube 112 and detachably securing a clip device 111 comprising distal tissue engaging arms 127 (with teeth 128), the arms 127 being movable between a radially expanded position 127' (in one embodiment a biased in the outward direction) and a closed position 127" and a proximal portion for detachably engaging the operating wire 114 and a sliding ring 132 in communication with the operating wire configured to close the clip arms 127 and grasp tissue. Optionally, the introducer tube 112 comprises an outer sheath 130 and an inner sheath 140. The assembly110 is passed through the endoscope working channel 74 such that the clip arms 127 are in a closed position until advancing to the protective cap's tissue receiving chamber 232 and expanding to the tissue receiving chamber's inner surface 236. Optionally, the introducer tube 112 of the assembly 110 for the clip device delivery apparatus is passed distally through the endoscope working channel 74 without the clip device 111, which is later attached to a second retainer 160 as described above.

The endoscope distal end portion 65 is positioned at the target site within the patient (step 340). The endoscope uses suction through the working channel or other channel to draw the target tissue into the protective cap receiving chamber 232 (step 350), illuminating the tissue with the light 72 and viewing the tissue through the lens 70.

The clip is deployed about the tissue (step 360). More particularly, holding the forward handle 116 relatively stationary, the gastroenterologist axially slides the rearward handle 118 distally so as to move the operating wire 114 and, therefore, the clip device 111 and sliding ring 132 detachably secured to the distal end of the operating wire. Using this technique, the gastroenterologist disposes the clip arms 127 into the cap's receiving chamber 232, and thereby allowing the arms 127 to expand to their radially expanded position 127' (in one embodiment a biased transverse outward direction) until constrained transversely by the cap's inner surface 236 and/or until inhibited (e.g., limited, controlled, and/or stopped, etc.) distally by the cap's optional flange 234, which the gastroenterologist will feel when forward movement of the handle 118 stops. Concomitantly, by moving the arms 127 into the receiving chamber 232, so too the gastroenterologist has moved the sliding ring 132 into the chamber 232. By moving the forward handle
116 distally (keeping the rearward handle 118 substantially stationary relative to the forward handle 116), the gastroenterologist may slide the sliding ring 132 over the clip arms 127 within the cap's chamber 232 until the clip arms 127 and teeth 128 close sufficiently to grasp the target tissue. Alternatively, the gastroenterologist may hold the forward handle 116 stationary while withdrawing the rearward handle 118 proximally, and because the sliding ring 132 abuts the clip stops 228, thereby preventing further proximal withdrawal of the sliding ring 132, the sliding ring 132 slides over and closes the clip arms 127 and teeth 128 into tissue engaging position. The gastroenterologist then may move the rearward handle 118, and therefore the distal end 114" of the operating wire 114, forward in order that second retainer 160 releases the first retainer 120 and the associated clip arms 127, thereby releasing the clip device 111 from the clip device delivery apparatus. By repeating these tissue engaging clip procedures with a second clip device 111, or with reloading a single-clip device or providing a different clip device, the gastroenterologist may deploy additional clips.

A method as taught herein (whether a method of assembling or using a clip device delivery apparatus) need not be performed sequentially. Nothing in this disclosure should be interpreted as imposing an order to the steps of the method(s). By way of example only and not by way of limitation, in method 300 the gastroenterologist may insert the endoscope distal end portion into a body cavity (step 340) and then insert a clip device through the endoscope working channel (step 330). Likewise, the gastroenterologist may suction tissue into the protective cap (step 350) prior to inserting the clip device through the endoscope working channel (step 330). Similarly, a gastroenterologist may insert the clip device into the endoscope working channel (step 330) and then attach the protective cap to a distal end portion of the endoscope (step 320). Furthermore, the gastroenterologist may deploy a clip (step 360) and then suction tissue into the protective cap (step 350).
Terms are to be given their reasonable plain and ordinary meaning.

## Claims

1. A clip device delivery apparatus for a gastrointestinal endoscope, comprising:
a haemostatic clip device (111) that is suitable to slideably fit through a working channel (74) of said elongate gastrointestinal endoscope (10), the clip device being detachably operatively coupled to an elongate deployment device (114), the clip device comprising at least three arms (127) movable between a radially expanded position (127') and a closed position (127");
**characterized in that** the clip delivery apparatus further comprises:
a detachable protective cap (210) comprising a proximal tube mounting section (220) that detachably fits over a distal end portion (65) of said gastrointestinal endoscope (10), the detachable protective cap (210) further comprising a distal section (230), the proximal tube mounting section (220) comprising a lumen (222) and the distal section (230) comprising a clip arm receiving chamber (232) configured to extend distal to said gastrointestinal endoscope distal end portion (65) and that is configured to accommodate radial expansion of the clip arms;
whereby the proximal tube mounting section lumen (222) is configured to detachably receive a distal end portion (65), (190') of said gastrointestinal endoscope (40), (112); and **in that** the clip delivery apparatus is configured so as to enable tissue to be suctioned into said clip arm receiving chamber and to enable the clip device to be deployed about said tissue within the clip arm receiving chamber.

2. The apparatus of claim 1, wherein the protective cap tube mounting section (220) comprises a proximal opening (224) and a tube engaging inner surface (226) configured to detachably operatively couple the protective cap tube mounting section (220) to said gastrointestinal endoscope distal end portion (65), (190').

3. The apparatus of claim 2 wherein the protective cap further comprises a tube stop (228) positioned intermediate the proximal tube mounting section proximal opening (224) and a distal opening (234) in said distal section, the tube stop (228) protruding inwardly toward the tube receiving lumen (228) and being sized to inhibit distal movement of said gastrointestinal endoscope distal end portion (65), (190') relative to the protective cap tube mounting section (220) and having a passageway (240) sized to allow passage of the clip arms (127) from the mounting section (220) to the clip arm receiving chamber (232).

4. The apparatus of claim 2 wherein the protective cap distal section (230) further comprises a flange (238) protruding inwardly toward the clip arm receiving chamber (232) and being sized to inhibit distal movement of at least the partially radially expanded clip arm (127) beyond the distal section distal opening (234).

5. The apparatus of claim 1 further comprising an elongate gastrointestinal endoscope (40), (112) having a working channel (74), (188) extending through a distal end portion (65), (190') thereof and configured to slideably receive the deployment device (114), the gastrointestinal endoscope distal end portion (65), (190') further comprising an outer surface (66), (196) and a distal end face (78), (198'), wherein the protective cap tube mounting section is detachably operatively coupled to the gastrointestinal endoscope distal end portion.

6. The apparatus of claim 5, wherein the protective cap tube mounting section fits over the gastrointestinal endoscope distal end portion (65) and is detachably mounted to the endoscope distal end portion outer surface.

7. The apparatus of claim 6 further comprising: an elongate outer sheath (130) slideably disposed within the gastrointestinal endoscope working channel (74) and having a distal end portion (190') and an outer sheath working channel (188); and an elongate inner sheath (140) slideably disposed within the outer sheath working channel and having a distal end portion (152) and an inner sheath working channel (148);
wherein said elongate deployment device (114) is an operating wire being substantially slideably received within the inner sheath working channel.

8. The apparatus of claim 5, further comprising an elongate outer sheath (130) having an outer sheath working channel (188), an outer sheath distal end portion (190'), and an outer sheath distal end portion outer surface (196) such that the protective cap tube mounting section is detachably mounted to the outer sheath distal end portion outer surface.

9. The apparatus of claim 8 further comprising an elongate inner sheath (140) having an inner sheath working channel (148), the inner sheath being slideably received within the outer sheath working channel and said elongate deployment device (114) is an operating wire being slideably within the inner sheath working channel.

10. The apparatus of claim 1, wherein the haemostatic clip device (111) further comprises a first retainer (120) with at least one of the arms (127) extending distally from the first retainer (120) having a distal tooth (128), and further comprises a sliding ring (132) having a lumen (138) configured to slideably receive the plurality of arms (127), the sliding ring (132) being positioned intermediate the first retainer (120) and the distal end tooth (127), the sliding ring (132) being configured to engage and close the arms (127) together.

11. The apparatus of claim 10 further comprising a second retainer (160) having a first end portion (162) detachably operatively coupled to the first retainer (120) and a second end portion (164) operatively coupled to the elongate deployment device (114).

12. The apparatus of claim 10 wherein the sliding ring (132) is movable between a sliding ring first position (135) disposed about a plurality of clip arms (127) to move the clip arms to a closed position (127") and a sliding ring second position (135') that allows the clip arms (127) to be spaced apart from each other in an expanded position (127').

13. The apparatus of claim 12 wherein the first retainer (120) comprises a distal first end (124), a proximal second end (122) comprising a hook (137) configured to engage a notch (166) of the second retainer (160), and a notch (126) configured to engage a hook (137') of the second retainer (160).

14. The apparatus of claim any preceding claim, wherein a tissue receiving section of said protective cap has a substantially conical structure.

15. The apparatus of claim 3, wherein said distal section includes only one distal opening, namely said distal opening.

16. The apparatus of claim 3 or claim 15, wherein said protective cap includes only one passageway between said proximal tube mounting section and said distal section, namely said passageway.

17. The apparatus of any one of claims 5 to 9, wherein said proximal tube mounting section lumen is configured to form an airtight engagement with said distal end portion of said gastrointestinal endoscope.

## Patentansprüche

1. Abgabevorrichtung für eine Klammervorrichtung für ein gastrointestinales Endoskop, umfassend:
eine hämostatische Klammervorrichtung (111), die geeignet ist, gleitend durch einen Arbeitskanal (74) des langgestreckten gastrointestinalen Endoskops (10) zu passen, wobei die Klammervorrichtung mit einer langgestreckten Einsatzvorrichtung (114) in lösbarer Wirkverbindung steht, wobei die Klammervorrichtung mindestens drei Arme (127) umfasst, die zwischen einer radial expandierten Position (127') und einer geschlossenen Position (127") bewegbar sind;
**dadurch gekennzeichnet, dass** die Klammerabgabevorrichtung ferner das Folgende umfasst:
eine abnehmbare Schutzkappe (210), die einen proximalen Schlauchbefestigungsabschnitt (220) aufweist, der lösbar über einen distalen Endabschnitt (65) des gastrointestinalen Endoskops (10) passt, wobei die abnehmbare Schutzkappe (210) ferner einen distalen Abschnitt (230) umfasst, der proximale Schlauchbefestigungsabschnitt (220) ein Lumen (222) umfasst und der distale Abschnitt (230) eine Klammerarmaufnahmekammer (232) umfasst, die dazu ausgelegt ist, sich distal zum distalen Endabschnitt (65) des gastrointestinalen Endoskops zu erstrecken und dazu ausgelegt ist, eine radiale Expansion der Klammerarme aufzunehmen;
wobei das Lumen (222) des proximalen Schlauchbefestigungsabschnitts dazu ausgelegt ist, einen distalen Endabschnitt (65), (190') des gastrointestinalen Endoskops (40), (112) lösbar aufzunehmen;
und dass die Klammerabgabevorrichtung derart ausgelegt ist, dass sie das Einsaugen von Gewebe in die Klammerarmaufnahmekammer ermöglicht und das Einsetzen der Klammervorrichtung um das Gewebe in der Klammerarmaufnahmekammer ermöglicht.

2. Vorrichtung nach Anspruch 1, wobei der Schlauchbefestigungsabschnitt (220) der Schutzkappe eine proximale Öffnung (224) und eine Schlaucheingriffsinnenseite (226) umfasst, die dazu ausgelegt ist, den Schlauchbefestigungsabschnitt (220) der Schutzkappe mit dem distalen Endabschnitt (65), (190') des gastrointestinalen Endoskops in lösbare Wirkverbindung zu bringen.

3. Vorrichtung nach Anspruch 2, wobei die Schutzkappe ferner einen Schlauchanschlag (228) umfasst, der zwischen der proximalen Öffnung (224) des proximalen Schlauchbefestigungsabschnitts und einer distalen Öffnung (234) im besagten distalen Abschnitt angeordnet ist, wobei der Schlauchanschlag (228) nach innen zum Schlauchaufnahmelumen (228) vorragt und eine Größe zur Unterdrückung einer distalen Bewegung des distalen Endabschnitts (65), (190') des gastrointestinalen Endoskops bezüglich des Schlauchbefestigungsabschnitts (220) der Schutzkappe sowie einen Durchgang (240) aufweist, der eine solche Größe hat, dass die Klammerarme (127) vom Befestigungsabschnitt (220) zur Klammerarmaufnahmekammer (232) durchgeleitet werden können.

4. Vorrichtung nach Anspruch 2, wobei der distale Abschnitt (230) der Schutzkappe ferner einen Flansch (238) umfasst, der nach innen zur Klammerarmaufnahmekammer (232) vorragt und eine Größe zur Unterdrückung einer distalen Bewegung zumindest des teilweise radial expandierten Klammerarms (127) bis hinter die distale Öffnung (234) des distalen Abschnitts aufweist.

5. Vorrichtung nach Anspruch 1, ferner umfassend ein langgestrecktes gastrointestinales Endoskop (40), (112) mit einem Arbeitskanal (74), (188), der sich durch einen distalen Endabschnitt (65), (190') davon erstreckt und zur gleitenden Aufnahme der Einsatzvorrichtung (114) ausgelegt ist, wobei der distale Endabschnitt (65), (190') des gastrointestinalen Endoskops ferner eine Außenfläche (66), (196) und eine distale Endfläche (78), (198') umfasst, wobei der Schlauchbefestigungsabschnitt der Schutzkappe mit dem distalen Endabschnitt des gastrointestinalen Endoskops in lösbarer Wirkverbindung steht.

6. Vorrichtung nach Anspruch 5, wobei der Schlauchbefestigungsabschnitt der Schutzkappe über den distalen Endabschnitt (65) des gastrointestinalen Endoskops passt und lösbar an der Außenfläche des distalen Endabschnitts des Endoskops befestigt ist.

7. Vorrichtung nach Anspruch 6, ferner umfassend: eine langgestreckte Außenhülle (130), die gleitend im Arbeitskanal (74) des gastrointestinalen Endoskops angeordnet ist und einen distalen Endabschnitt (190') und einen Außenhüllenarbeitskanal (188) aufweist; und eine langgestreckte Innenhülle (140) die gleitend im Außenhüllenarbeitskanal angeordnet ist und einen distalen Endabschnitt (152) und einen Innenhüllenarbeitskanal (148) aufweist;
wobei die langgestreckte Einsatzvorrichtung (114) ein Betätigungsdraht ist, der im Wesentlichen gleitend im Innenhüllenarbeitskanal aufgenommen ist.

8. Vorrichtung nach Anspruch 5, ferner umfassend eine langgestreckte Außenhülle (130) mit einem Außenhüllenarbeitskanal (188), einem distalen Außenhüllen-Endabschnitt (190') und einer Außenfläche (196) des distalen Außenhüllen-Endabschnitts, derart, dass der Schlauchbefestigungsabschnitt der Schutzkappe lösbar an der Außenfläche des distalen Außenhüllen-Endabschnitts befestigt ist.

9. Vorrichtung nach Anspruch 8, ferner umfassend eine langgestreckte Innenhülle (140) mit einem Innenhüllenarbeitskanal (148), wobei die Innenhülle gleitend im Außenhüllenarbeitskanal aufgenommen ist und die langgestreckte Einsatzvorrichtung (114) ein Betätigungsdraht ist, der gleitend im Innenhüllenarbeitskanal angeordnet ist.

10. Vorrichtung nach Anspruch 1, wobei die hämostatische Klammervorrichtung (111) ferner eine erste Haltevorrichtung (120) umfasst, wobei mindestens einer der Arme (127), der sich distal von der ersten Haltevorrichtung (120) erstreckt, einen distalen Zahn (128) aufweist, und wobei sie ferner einen Gleitring (132) mit einem Lumen (138) umfasst, das zur gleitenden Aufnahme der Vielzahl von Armen (127) ausgelegt ist, wobei der Gleitring (132) zwischen der ersten Haltevorrichtung (120) und dem distalen Endzahn (127) angeordnet ist, wobei der Gleitring (132) ausgelegt ist, die Arme (127) in Eingriff zu nehmen und zusammen zu schließen.

11. Vorrichtung nach Anspruch 10, ferner umfassend eine zweite Haltevorrichtung (160) mit einem ersten Endabschnitt (162), der mit der ersten Haltevorrichtung (120) in lösbarer Wirkverbindung steht, und mit einem zweiten Endabschnitt (164), der mit der langgestreckten Einsatzvorrichtung (114) in Wirkverbindung steht.

12. Vorrichtung nach Anspruch 10, wobei der Gleitring (132) zwischen einer ersten Gleitringposition (135), die um eine Vielzahl von Klammerarmen (127) angeordnet ist, um die Klammerarme in eine geschlossene Position (127") zu bewegen, und einer zweiten Gleitringposition (135'), in der die Klammerarme (127) in Abstand voneinander in eine expandierte Position (127') gebracht werden können, bewegbar ist.

13. Vorrichtung nach Anspruch 12, wobei die erste Haltevorrichtung (120) ein distales erstes Ende (124), ein proximales zweites Ende (122), das einen für einen Eingriff in eine Kerbe (166) der zweiten Haltevorrichtung (160) ausgelegten Haken (137) umfasst, und eine für einen Eingriff mit einem Haken (137') der zweiten Haltevorrichtung (160) ausgelegte Kerbe (126) umfasst.

14. Vorrichtung nach einem der vorhergehenden Ansprüche, wobei ein Gewebeaufnahmeabschnitt der Schutzkappe eine im Wesentlichen konische Struktur aufweist.

15. Vorrichtung nach Anspruch 3, wobei der distale Abschnitt nur eine distale Öffnung, nämlich die besagte distale Öffnung aufweist.

16. Vorrichtung nach Anspruch 3 oder Anspruch 15, wobei die Schutzkappe nur einen Durchgang zwischen dem proximalen Schlauchbefestigungsabschnitt und dem distalen Abschnitt, nämlich den besagten Durchgang aufweist.

17. Vorrichtung nach einem der Ansprüche 5 bis 9, wobei das Lumen des proximalen Schlauchbefestigungsabschnitts zur Bildung eines luftdichten Eingriffs mit dem distalen Endabschnitt des gastrointestinalen Endoskops ausgelegt ist.

## Revendications

1. Appareil de pose de dispositif de clip pour un endoscope gastrointestinal, comprenant :
un dispositif de clip hémostatique (111) qui est apte à s'adapter à coulissement à travers un canal opérateur (74) dudit endoscope gastrointestinal (10) allongé, le dispositif de clip étant accouplé, de manière fonctionnelle et de manière amovible, avec un dispositif de mise en place (114) allongé, le dispositif de clip comprenant au moins trois bras (127) mobiles entre une position déployée radialement (127') et une position fermée (127") ;
**caractérisé en ce que** l'appareil de pose de clip comprend en outre :
une coiffe protectrice amovible (210) comprenant une section d'assemblage de tube proximale (220) qui s'adapte de manière amovible sur une partie d'extrémité distale (65) dudit endoscope gastrointestinal (10), la coiffe protectrice amovible (210) comprenant en outre une section distale (230), la section d'assemblage de tube proximale (220) comprenant une lumière (222) et la section distale (230) comprenant une chambre de réception de bras de clip (232) configurée pour s'étendre dans une direction distale vis-à-vis de ladite partie d'extrémité distale (65) dudit endoscope gastrointestinal et qui est configurée pour permettre un déploiement radial des bras de clip ;
la lumière (222) de la section d'assemblage de tube proximale (222) étant configurée pour recevoir de manière amovible une partie d'extrémité distale (65), (190') dudit endoscope gastrointestinal (40), (112) ;
et **en ce que** l'appareil de pose de clip est configuré de façon à permettre une aspiration du tissu dans ladite chambre de réception de bras de clip et à permettre un déploiement du dispositif de clip autour dudit tissu à l'intérieur de la chambre de réception de bras de clip.

2. Appareil selon la revendication 1, dans lequel la section d'assemblage de tube (220) de la coiffe protectrice comprend une ouverture proximale (224) et une surface intérieure destinée à venir en prise avec le tube (226) configurée pour accoupler, de manière fonctionnelle et de manière amovible, la section d'assemblage de tube (220) de la coiffe protectrice avec ladite partie d'extrémité distale (65), (190') dudit endoscope gastrointestinal.

3. Appareil selon la revendication 2, dans lequel la coiffe protectrice comprend en outre une butée de tube (228) positionnée entre l'ouverture proximale (224) de la section de montage de tube proximale et une ouverture distale (234) dans ladite section distale, la butée de tube (228) faisant saillie vers l'intérieur en direction de la lumière de réception de tube (228) et étant dimensionnée de façon à empêcher un déplacement dans la direction distale de ladite partie d'extrémité distale (65), (190') dudit endoscope gastrointestinal par rapport à la section d'assemblage de tube (220) de la coiffe protectrice et comportant un passage (240) dimensionné pour permettre le passage des bras de clip (127) de la section d'assemblage (220) à la chambre de réception de bras de clip (232).

4. Appareil selon la revendication 2, dans lequel la section distale (230) de la coiffe protectrice comprend en outre une collerette (238) faisant saillie vers l'intérieur en direction de la chambre de réception de bras de clip (232) et étant dimensionnée de façon à empêcher un déplacement dans la direction distale au moins du bras de clip (127) partiellement déployé radialement au-delà de l'ouverture distale (234) de la section distale.

5. Appareil selon la revendication 1, comprenant en outre un endoscope gastrointestinal (40), (112) allongé comportant un canal opérateur (74), (188) s'étendant à travers une partie d'extrémité distale (65), (190') de celui-ci et configuré pour recevoir à coulissement le dispositif de mise en place (114), la partie d'extrémité distale (65), (190') de l'endoscope gastrointestinal comprenant en outre une surface extérieure (66), (196) et une face d'extrémité distale (78), (198'), dans lequel la section d'assemblage de tube de la coiffe protectrice est accouplée, de manière fonctionnelle et de manière amovible, à la partie d'extrémité distale de l'endoscope gastrointestinal.

6. Appareil selon la revendication 5, dans lequel la section d'assemblage de tube de la coiffe protectrice s'adapte sur la partie d'extrémité distale (65) de l'endoscope gastrointestinal et est assemblée de manière amovible sur la surface extérieure de la partie d'extrémité distale de l'endoscope.

7. Appareil selon la revendication 6, comprenant en outre : une gaine extérieure (130) allongée disposée à coulissement à l'intérieur du canal opérateur (74) de l'endoscope gastrointestinal et comportant une partie d'extrémité distale (190') et un canal opérateur (188) de gaine extérieure ; et une gaine intérieure (140) allongée disposée à coulissement à l'intérieur du canal opérateur de gaine extérieure et comportant une partie d'extrémité distale (152) et un canal opérateur (148) de gaine intérieure ;
dans lequel ledit dispositif de mise en place (114) allongé est un fil de manipulation qui est reçu essentiellement à coulissement à l'intérieur du canal opérateur de gaine intérieure.

8. Appareil selon la revendication 5, comprenant en outre une gaine extérieure (130) allongée comportant un canal opérateur (188) de gaine extérieure, une partie d'extrémité distale (190') de gaine extérieure et une surface extérieure (196) de partie d'extrémité distale de gaine extérieure telle que la section d'assemblage de tube de la coiffe protectrice soit assemblée, de manière amovible, sur la surface extérieure de partie d'extrémité distale de gaine extérieure.

9. Appareil selon la revendication 8, comprenant en outre une gaine intérieure (140) allongée comportant un canal opérateur (148) de gaine intérieure, la gaine intérieure étant reçue à coulissement à l'intérieur du canal opérateur de gaine extérieure et ledit dispositif de mise en place (114) allongé est un fil de manipulation pouvant coulisser à l'intérieur du canal opérateur de gaine intérieure.

10. Appareil selon la revendication 1, dans lequel le dispositif de clip hémostatique (111) comprend en outre un premier élément de retenue (120), au moins un des bras (127) s'étendant dans la direction distale à partir du premier élément de retenue (120) comportant une dent distale (128), et comprend en outre une bague coulissante (132) comportant une lumière (138) configurée pour recevoir à coulissement la pluralité de bras (127), la bague coulissante (132) étant positionnée entre le premier élément de retenue (120) et la dent d'extrémité distale (127), la bague coulissante (132) étant configurée pour venir en prise avec les bras (127) et les rassembler en position fermée.

11. Appareil selon la revendication 10, comprenant en outre un second élément de retenue (160) comportant une première partie d'extrémité (162) accouplée, de manière fonctionnelle et de manière amovible, avec le premier élément de retenue (120) et une seconde partie d'extrémité (164) accouplée, de manière fonctionnelle, avec le dispositif de mise en place (114) allongé.

12. Appareil selon la revendication 10, dans lequel la bague coulissante (132) est mobile entre une première position de bague coulissante (135) disposée autour d'une pluralité de bras de clip (127) de façon à déplacer les bras de clip jusqu'à la position fermée (127") et une seconde position de bague coulissante (135') qui permet aux bras de clip (127) d'être espacés les uns des autres dans une position déployée (127').

13. Appareil selon la revendication 12, dans lequel le premier élément de retenue (120) comprend une première extrémité distale (124), une seconde extrémité proximale (122) comprenant un crochet (137) configuré pour venir en prise avec un cran (166) du second élément de retenue (160), et un cran (126) configuré pour venir en prise avec un crochet (137') du second élément de retenue (160).

14. Appareil selon l'une quelconque des revendications précédentes, dans lequel une section de réception de tissu de ladite coiffe protectrice présente une structure essentiellement conique.

15. Appareil selon la revendication 3, dans lequel ladite section distale comprend une seule ouverture distale, à savoir ladite ouverture distale.

16. Appareil selon la revendication 3 ou la revendication 15, dans lequel ladite coiffe protectrice comprend un seul passage entre ladite section d'assemblage de tube proximale et ladite section distale, à savoir ledit passage.

17. Appareil selon l'une quelconque des revendications 5 à 9, dans lequel ladite lumière de ladite section d'assemblage de tube proximale est configurée pour venir en prise de manière étanche avec ladite partie d'extrémité distale dudit endoscope gastrointestinal.
